Europäisches Patentamt

(19) European Patent Office

Office européen des brevets

(11) Publication number: **0 158 179**
**B1**

(12) **EUROPEAN PATENT SPECIFICATION**

(45) Date of publication of patent specification: **19.09.90**

(21) Application number: **85103255.7**

(22) Date of filing: **20.03.85**

(51) Int. Cl.⁵: **C 07 G 11/00,** C 12 P 1/06,
A 23 K 1/17, C 12 N 1/20 //
(C12P1/06, C12R1:04,
C12N1:20, C12R1:04)

(54) Antibiotic 6270, process for its production, and its use as an anticoccidiosis agent and a feed additive.

(30) Priority: **13.04.84 JP 72912/84**

(43) Date of publication of application:
**16.10.85 Bulletin 85/42**

(45) Publication of the grant of the patent:
**19.09.90 Bulletin 90/38**

(84) Designated Contracting States:
**AT BE CH DE FR GB IT LI LU NL SE**

(56) References cited:

CHEMICAL ABSTRACTS, vol. 99, 1983, page 506,
abstract no. 120681p, Columbus, Ohio, US; &
JP-A-58 86 087 (MEIJI SEIKA KAISHA LTD)
23-05-1983

CHEMICAL ABSTRACTS, vol. 103, 1985, page
409, abstract no. 36179z, Columbus, Ohio, US; &
JP-A-60 41 489 (KYOWA HAKKO KOGYO CO.
LTD) 05-03-1985

(73) Proprietor: **KAKEN PHARMACEUTICAL CO.,
LTD.**
**No. 28-8, 2-chome, Honkomagome Bunkyo-Ku
Tokyo 113 (JP)**

(72) Inventor: **Kusakabe, Yoko Kaken
Pharmaceutical Co. Ltd.**
**Tokyo Kenkyusho 1-6-42, Jujodai
Kita-ku Tokyo (JP)**
Inventor: **Takahashi, Nobuko Kaken
Pharmaceutical Co. Ltd.**
**Tokyo Kenkyusho 1-6-42, Jujodai
Kita-ku Tokyo (JP)**
Inventor: **Seino, Akio Kaken Pharmaceutical Co.
Ltd.**
**Tokyo Kenkyusho 1-6-42, Jujodai
Kita-ku Tokyo (JP)**
Inventor: **Iwagaya, Yasuo Kaken
Pharmaceutical Co. Ltd.**
**Shizuoka Kojyo, 301, Gensuke
Fujieda-shi, Shizuoka-ken (JP)**

(74) Representative: **Wächtershäuser, Günter, Dr.**
**Tal 29**
**D-8000 München 2 (DE)**

**Description**

The present invention relates to a novel antibiotic 6270, a process for its production, and its use as an anticoccidiosis agent or as a feed additive to accelerate the growth of domestic animals or fowls.

The present inventors have isolated a number of microorganisms from various soils for the purpose of searching new antibiotics and studied the antibiotics produced by the isolated microorganisms. As a result, they have found that when a microorganism belonging to the genus *Nocardiopsis* isolated from soil collected in Abashiri city, Hokkaido, Japan, is cultured in a proper culture medium, an antibiotic having a high antibacterial activity against gram positive bacteria is accumulated in the culture medium. This antibiotic was isolated and examined for the physicochemical properties and biological properties, whereby it has been confirmed to be a new antibiotic and named an antibiotic 6270.

The present invention is based on this discovery, and provides the new antibiotic 6270 and a process for its production which comprises culturing an antibiotic 6270-producing microorganism belonging to the genus *Nocardiopsis* and isolating the antibiotic 6270 from the culture product.

Further, the present invention provides an anticoccidiosis agent which contains the antibiotic 6270 as the active ingredient, and a method for preventing and curing coccidiosis of domestic fowls and animals which comprises administering an effective amount of the antibiotic 6270 to the domestic fowls and animals.

Furthermore, the present invention provides a growth accelerating and feed efficiency increasing agent for domestic animals and fowls, which comprises the antibiotic 6270 as the effective ingredient, and a method for accelerating the growth of domestic animals and fowls and increasing the feed efficiency thereof, which comprises administering an effective amount of the antibiotic 6270 to the animals and fowls. Accordingly, the present invention provides a feed of domestic animals and fowls, which contains an effective amount of the antibiotic 6270. The effective amount in this respect is usually from 0.5 to 200 ppm, preferably from 1 to 100 ppm.

Now, the present invention will be described in detail with reference to the preferred embodiments.

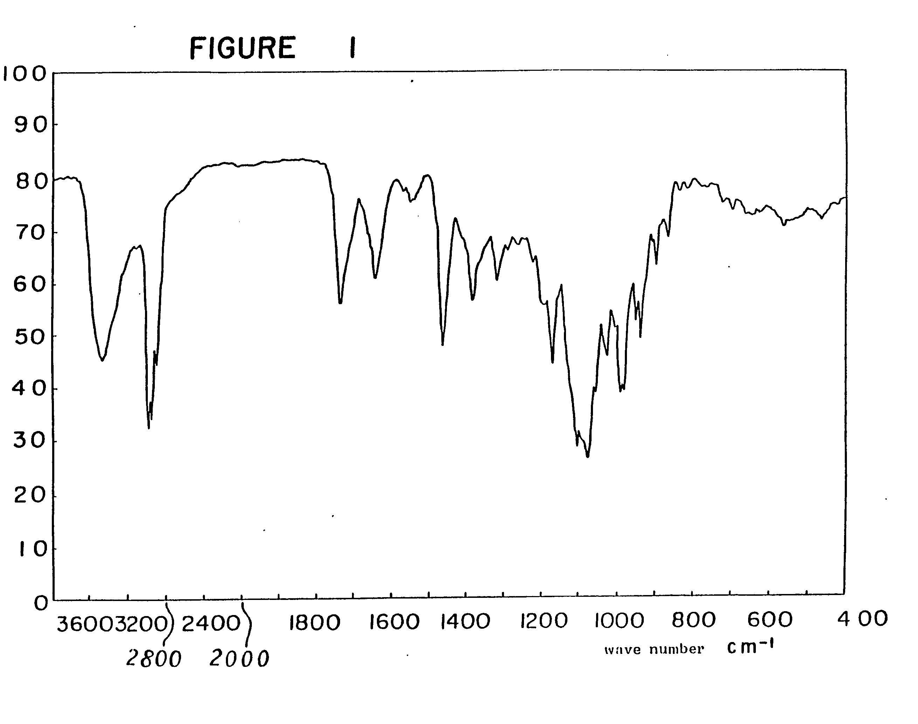

In the accompanying drawings, Figure 1 is the infrared absorption spectrum of the antibiotic 6270 as measured by means of potassium bromide tablet.

Figure 2 is the $^1$H-NMR spectrum of the antibiotic 6270 as measured in heavy chloroform by using TMS as internal standard.

Figure 3 is the $^{13}$C-NMR spectrum of the antibiotic 6270 as measured in heavy chloroform by using TMS as internal standard.

The microorganism which may be employed for the production of the antibiotic 6270, may be any microorganism belonging to genus *Nocardiopsis* so long as it is capable of producing the antibiotic 6270. However, it is preferred to employ *Nocardiopsis* sp. 6270 strain isolated afresh by the present inventors (deposited in Fermentation Research Institute of Japan as FERM BP-717).

The *Nocardiopsis* sp. 6270 strain used in the present invention has branched mycelia, and aerial mycelia extending in air are fragmented to constitute long spore chains. From the hydrolyzate of the whole cells, meso-diaminopimeric acid, and galactose and ribose as sugar components are detected. However, arabinose and madurose are not detected. Further, mycolic acid is not detected. The menaquinone composition comprises MK-9 (H$_6$) and (H$_8$) as the main components and MK-9 (H$_4$) was detected as a trace component. The width of substratal mycelium is about 0.5 µm. From the foregoing characteristics, the 6270 strain is determined to be a strain belonging to genus *Nocardiopsis* among Actinomyces. The *Norcardiopsis* sp. 6270 strain has the following microbiological properties.

I. Morphological characteristics

The substratal mycelia are well developed and branched, whereby the width of each mycelium is about 0.4 µm. Aerial mycelia grow well in some culture media such as a sucrose-nitrate agar culture medium (Difco. Czapeck's Solution Agar), a starch agar culture medium (ISP-4, Difco-Inorganic Salt Starch Agar), an oatmeal agar culture medium (ISP-8) or a glucose-asparagine agar culture medium. The aerial mycelia are straight or curved, and they constitute a cobweb as a whole. When matured, the aerial mycelia have a width of from 0.4 to 0.6 µm. Upon expiration of 10 days from the initiation of the cultivation, the aerial mycelia undergo fragmentation and form long spore chains each comprising at least 10 spores. The spore chains are not spiral. The surface of the spores are smooth, and the shape is cylindrical. The size is about 0.4×1.2 µm. No sporangium, sclerotium or synnema was observed.

II. Cultural characteristics

The tests were conducted in accordance with the test method reported by E. B. Shirling et al. (International Journal of Systematic Bacteriology, *16*, 313—340 (1966)). Additionally, known culture media and test methods were employed.

The color was determined under the standard light source of xenon lump by using Color Harmony Manual, 4th edition, 1958 as the color standards. When the corresponding color tab was found, it was shown by the common names first, and then the color tab code was shown in parenthesis. When the same color appeared repeatedly, only the color tab code was indicated.

Unless otherwise specified, the following data represent the growth condition as cultured for three weeks at 28°C on an agar plate culture medium. Item (a) indicates the growth; item (b) indicates the state of aerial mycelia; item (c) indicates a soluble pigment; and item (d) presents other characteristics.

(1) Sucrose-nitrate culture medium (Difco-Czapeck's Solution Agar)

a: Good, near gray (2ba)
b: White, powdery
c: None

(2) Glucose-asparagine culture medium

a: Good, light amber (3ic) to beige (3ge)
b: Near gray (3ba)
c: None

(3) Glycerol-asparagine culture medium

a: No good, 2ba
b: Slightly forms
c: None

(4) Starch agar culture medium

a: Excellent, 3ic to Shale (3ca)
b: Powdery, white
c: None
d: Hydrolysis of starch: negative

(5) Tyrosine agar culture medium

a: No good, 3ge
b: None
c: None

(6) Nutrient agar medium (Difco. Bacto Nutrient Agar)

a: No good, light wheat (2ea)
b: None
c: None

(7) Yeast-malt extract agar culture medium

a: Excellent, cracks, topaz (3ne)
b: White
c: None

(8) Oatmeal culture medium

a: Good, shell (2ca)
b: 2ba
c: None

III. Physiological characteristics

(1) Growth temperature range (Slanted starch agar culture medium, temperature gradient incubator): 6.5—38°C (No growth observed below 5°C or above 39.5°C) Optimum temperature: 27°C—38°C

(2) Liquefaction of glucose-peptone-gelatin stab culture: Positive

(3) Hydrolysis of starch: Negative

(4) Coagulation and peptonization skim milk: No coagulation, and substantial peptonization (37°C)

(5) Formation of melanine:
ISP-tyrosine agar culture medium: Negative
ISP-triptone-yeast extract broth culture medium: Negative
ISP-peptone-yeast-iron agar culture medium: Negative

(6) NaCl tolerance: No growth at 4% or higher

(7) Solubility of xanthin, hypoxanthin, adenin: Negative

IV. Carbon source utilization (Pridham and Gottlieb basal medium)
Positive: D-glucose, L-arabinose, D-xylose, salicin
Weakly positive: D-fructose, sucrose
Negative: Inositol, L-rhamnose, raffinose, D-mannitol

The 6270 strain has been identified to be a strain belonging to *Nocardiopsis* Meyer 1976 among Actinomyces, since among the above-mentioned various characteristics, it forms long spore chains on the aerial mycelia, and the whole cell hydrolizate contains meso-diaminopimeric acid and galactose, but does not contain arabinose and madurose, and further no mycolic acid is detected.

In carrying out the process of the present invention, the antibiotic 6270-producing microorganism may be cultured in accordance with the conventional methods for cultivation of known actinomyces. However, from the industrial viewpoint, it is advantageous to employ a method of cultivation with aeration-agitation. As the culture medium for the production, there may be employed materials which are commonly used for the cultivation of actinomyces. Namely, various carbon sources, nitrogen sources and organic or inorganic salts, as well as a defoaming agent, if necessary, may be used in a suitable combination. For instance, as the carbon sources, there may be used glucose, starch, glycerol, dextrin, sucrose and animal or vegetable oils. As the nitrogen sources, there may be employed soybean meal, corn steep liquor, wheat embryo, and ammonia. Further, if necessary, inorganic salts such as calcium carbonate, sodium chloride, potassium chloride, and phosphates may be added. It is also possible to add organic or inorganic salts having an action to assist the growth of the microorganism and thereby facilitate the production of the antibiotic 6270. The temperature for the cultivation is usually within a range of from 25 to 35°C, preferably around 30°C. The cultivation time may vary depending upon the various conditions. However, usually the accumulation of the produced antibiotic 6270 reaches to the maximum within from 72 to 148 hours.

The antibiotic 6270 may be isolated from the culture medium and purified by utilizing its physicochemical properties by means of conventional methods such as a method of utilizing the difference in the solubility from impurities, a method of utilizing the difference in the adsorption to ion exchange resins or various adsorbing agents, a method of extracting with an organic solvent immiscible with water, or a proper combination of various means such as precipitation, removal of impurities, dialysis, drying and recrystallization.

For instance, the antibiotic 6270 produced in the culture medium may preferably be recovered as follows. After addition of a filter aid such as diatomaceous earth or Radiolite 700, the culture broth is filtered, and the filtrate and the microbial cells are respectively extracted with suitable solvents such as ethyl acetate and acetone. Then, the cell extract and the filtrate extract are combined. The solvent is distilled off from this extract solution, whereby the antibiotic 6270 is obtained as crude crystals. In order to isolate the antibiotic 6270 from the crude crystals, the crude crystals are subjected to, for example, chromatography. The eluate is concentrated under reduced pressure, and the residue is dissolved in a suitable solvent such as ethyl acetate and then treated with a dilute hydrochloric acid. The solvent layer is concentrated, whereby the antibiotic 6270 crystallizes as free acid.

In order to obtain a sodium salt, the above-mentioned solvent layer treated with a dilute hydrochloric acid solution, is treated with a dilute sodium carbonate solution, and then the solvent layer is concentrated, whereby the antibiotic 6270 is obtainable in the form of a sodium salt. The free acid-type or sodium salt-type antibiotic 6270 thus obtained can be recrystallized from a suitable solvent such as n-hexane-ethylacetate to obtain pure crystals. The free acid-type antibiotic 6270 thus obtained has the following properties:

(1) Colorless needle-like crystals; acidic substance

(2) Melting point: 115—118°C

(3) Elementary analysis (Found %): C: 62.72%, H: 9.49%; O: 27.78%

(4) Specific rotation: $[\alpha]_D^{20}$—11.5° (C 1.0, methanol)

(5) Ultraviolet absorption spectrum: The maximum absorption bands not observed at 210 nm or more, as measured in the methanol solution

(6) Characteristic absorption ($cm^{-1}$) in the infrared absorption spectrum (taken with the potassium bromide tablet): 3470, 2980, 2945, 2890, 1735, 1460, 1380, 1315, 1165, 1102, 1075, 987, 980. (The absorption spectrum is shown in Figure 1).

(7) Solubility: Soluble in methanol ethanol, ethyl acetate, chloroform, ether, acetone, and benzene; insoluble in water

(8) Color reactions: Positive in the vanilline-sulfuric acid reaction and the Dragendorff reaction; negative in the ninhydrin reaction; colors with $I_2$ gas

(9) Thin layer chromatography (by means of Kieselgel GF$_{254}$, manufactured by Merck Co.):

| Solvent system | Rf value |
| --- | --- |
| Chloroform-methanol (20:1) | 0.49 |
| n-Hexane-ethyl acetate (1:1) | 0.08 |
| Benzene-ethyl acetate (1:1) | 0.04 |
| Benzene-acetone (1:1) | 0.53 |
| Chloroform-acetone (2:1) | 0.36 |
| n-Hexane-ethyl acetate (1:3) | 0.13 |
| Ethyl acetate | 0.22 |

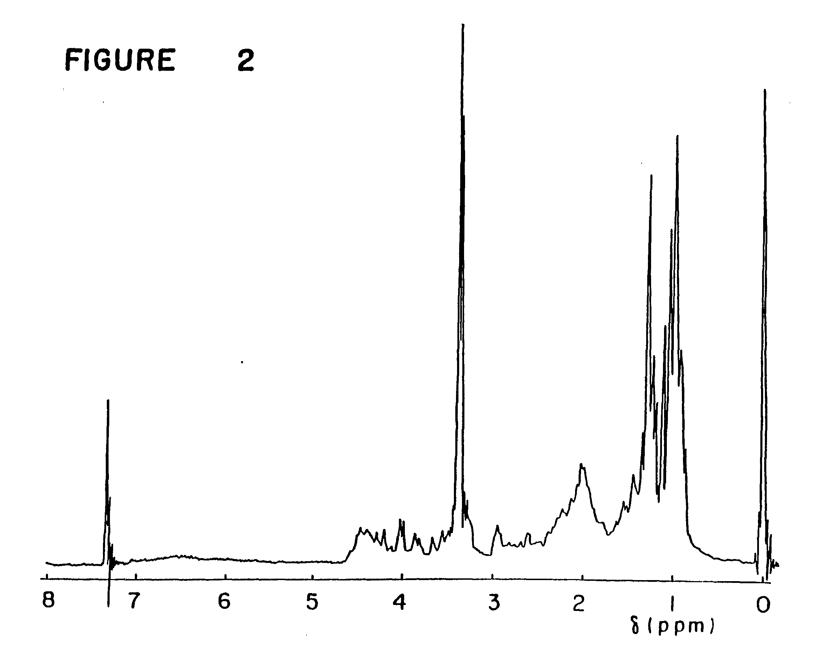

(10) $^1$H-NMR spectrum: The presence of three methoxy groups was observed at δppm 3.39 (3H), 3.49 (6H), as measured at 100 MHz in heavy chloroform by using TMS as internal standard (The spectrum is shown in Figure 2).

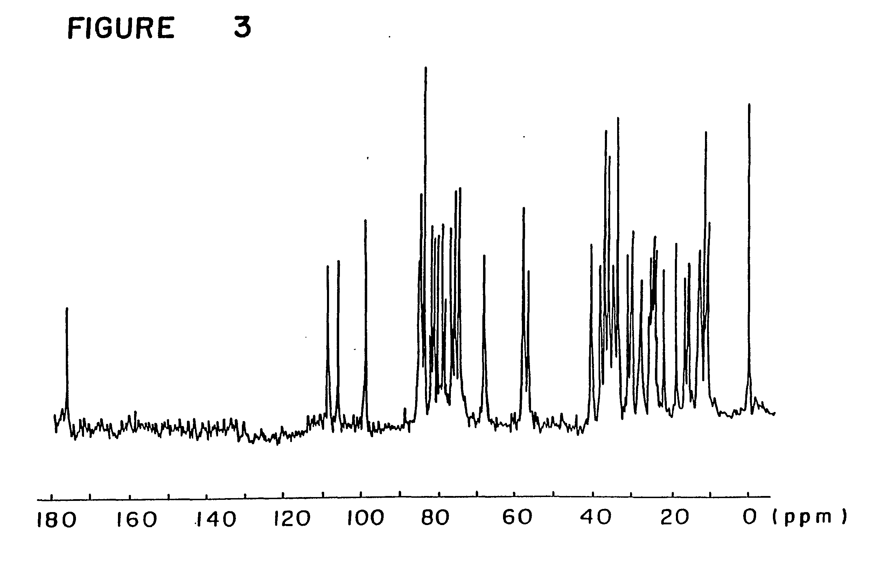

(11) $^{13}$C-NMR spectrum: the results of the measurement at 25 MHz in heavy chloroform by using TMS as internal standard, are shown in Figure 3. According to this result, the total number of carbon atoms is 44.

(12) The antibacterial spectrum is shown in Table 1.

4

TABLE 1

| Test microorganism | Minimum inhibitory concentration (mcg/ml) | Culture medium |
|---|---|---|
| Bacillus cereus IAM-1729 | 1.56 | a |
| Bacillus circulans IFO 3329 | 1.56 | a |
| Bacillus subtilis PCI 219 | 6.25 | a |
| Micrococcus flavus IFO 3242 | 1.56 | a |
| Sarcina lutea NIHJ | 3.13 | a |
| Staphylococcus aureus FDA209P JC-1 | 6.25 | a |
| Staphylococcus aureus (resistant to penicillin, streptomycin, chloramphenicol, tetracycline and kanamycin) | 6.25 | a |
| Escherichia coli NIHJ JC-2 | >100 | a |
| Escherichia coli (resistant to streptomycin, kanamycin, chloramphenicol, and tetracycline) | >100 | a |
| Klebsiella pneumoniae PCI 602 | >100 | a |
| Proteus vulgaris OX-19 | >100 | a |
| Pseudomonas aeruginosa IFO-3756 | >100 | a |
| Mycobacterium smegmatis ATCC 607 | 1.56 | b |
| Mycobacterium avium IFO 3153 | 1.56 | b |
| Mycobacterium phlei NIHJ | 3.13 | b |
| Penicillium chrysogenum Q-176 | >100 | c |
| Aspergillus flavus ATCC 9643 | >100 | c |
| Cochliobolus miyabeanus | 12.5 | c |
| Alternaria mari | 50 | c |
| Candida albicans YU 1200 | >100 | c |
| Saccharomyces cerevisiae Strain 77 | >100 | c |

Notes:
   a: Nutrient agar culture medium
   b: Glycerol nutrient agar culture medium
   c: Potato-sucrose agar culture medium

From the foregoing physicochemical and biological characteristics, this substance has been confirmed to belong to a general class of antibiotics called polyethers.

This substance is assumed to have three methoxy groups in a molecule from the $^1$H-NMR spectrum. As known antibiotics having three methoxy groups in one molecule, among the polyether antibiotics, there may be mentioned C20-12 (Japanese Unexamined Patent Publication No. 53919/1983), A-204B (Handbook

5

of Microbiology 410, vol. 3, CRC Press), T-42082 (Japanese Unexamined Patent Publication No. 79730/1976), T-40517 (Japanese Unexamined Patent Publication No. 10581/1975), 38295 (Japanese Unexamined Patent Publication No. 125793/1976), No. 6016 (Japanese Unexamined Patent Publication No. 84576/1979), X-14868C (Japanese Unexamined Patent Publication No. 120696/1981), CP-47433 (Japanese Unexamined Patent Publication No. 154108/1982), 47434 (Japanese Unexamined Patent Publication No. 154187/1982) and LL-C23024β (Japanese Unexamined Patent Publication No. 78598/1983).

However, the 6270 antibiotic differs from these known substances in the characteristics such as the melting point, specific rotation, elementary analytical values or infrared absorption spectrum, and is apparently a new antibiotic.

The antibiotic 6270 has particularly strong antibacterial effects against gram-positive bacteria, and is useful as an antibacterial agent.

Further, it has anticoccidiosis, acaricidal and antiviral activities at a low concentration, and it is useful as anticoccidiosis agent of domestic fowls such as chicken, quail, duck, goose and turkey, and domestic animals such as cattle, swine, rabbit, buffalo, goat and sheep, as an agent for treating diarrhoea of domestic animals, or as an agent to increase feed efficiency or to accelerate the growth of domestic animals and fowls.

Now, the present invention will be described in further detail with reference to Examples. However, it should be understood that the present invention is by no means restricted to these specific Examples.

Example 1

*Nocardiopsis* sp. 6270 strain (FERM BP-717) was inoculated to 1 liter of a culture medium (pH 6.0) comprising 6.0% of glucose, 2.0% of soybean meal, 1.0% of torula yeast and 0.5% of calcium carbonate, and cultivated at 30°C for 48 hours. This culture broth was inoculated to 100 liters of a culture medium having the same composition as above, and cultured at 30°C for 96 hours under stirring and aeration in a tank having a capacity of 200 liters. The rate of aeration was 100 liters per minute, and the rotation of the stirrer was 250 rpm. After addition of a filter aid (Radiolite 700, trademark) this culture broth was filtered to separate the filtrate and cells. Then, the filtrate was extracted with 40 liters of ethyl acetate, and the cells were extracted with 30 liters of acetone. The acetone extract of the cells was concentrated under reduced pressure to remove acetone, and extracted with 20 liters of ethyl acetate. This extract was combined with the extract from the filtrate, and the combined solution was concentrated under reduced pressure. The residue was adsorbed on a column of 750 g of silica gel (Wako gel C-200, tradename) packed with chloroform, and a mixture of chloroform and methanol (100:1) was passed through the column. The active fraction was concentrated under reduced pressure to dryness, whereby an oily residue was obtained. Then, the residue was dissolved in a small amount of acetone, and the solution was adsorbed on a column of Sephadex LH-20 filled with acetone, and eluted with acetone. The active fraction thereby obtained was concentrated to obtain crude crystals of the antibiotic 6270. The crystals were dissolved in ethyl acetate, and shaked with dilute hydrochloric acid. The ethyl acetate layer was concentrated under reduced pressure. The formed crystals are recrystallized from n-hexane-ethylacetate, whereby 23.4 g of free acid of the antibiotic 6270 was obtained as colorless needle-like crystals. The free acid of the antibiotic 6270 thus obtained exhibited the above-mentioned physicochemical and biological characteristics.

Test Example 1: Anticoccidiosis activity

Tested agent and feed:

The antibiotic 6270 was uniformly mixed with a perfect combination feed for chicks (manufactured by Oriental Yeast Co.) to obtain a predetermined concentration. The feed thus obtained was freely taken by chickens from two days before the inoculation of oocyst to the end of the test (eight days after the infection).

As a comparative agent, Monensin was used which is known to be an anticoccidiosis agent.

Chickens used:

Chickens used in this test were healthy cocks of egg-laying fowl (Shever Starcross) which were 7 days old (9 days old when infected) and had been bred under the conditions of perfect prevention of coccidiosis infection. Every group had five chickens. Oocyst inoculated and the quantity of inoculation:

The oocyst used for the infection was a sensitive strain of *Eimeria tenella.* Every chicken was inoculated to the crop with full grown oocysts ($3 \times 10^4$), orally by using metal zonde.

Judgement of effect:

The effect of the agents was determined by the Anticoccidial Index (ACI) which was calculated by the following formula:

ACI=(Relative increase in weight+Survival rate) −(Oocyst value+Disease value)

120 or less: Not effective as an anticoccidiosis agent.

160 or less: Slightly effective as an anticoccidiosis agent.

160—180: Moderately effective as an anticoccidiosis agent.

180 or higher: Extremely effective as an anticoccidiosis agent.

(i) Relative increase in weight

At the end of the test, the increase in weight (i.e. the weight at the end of the test—the weight at the time of infection) of each test group was measured and the relative increase in weight was calculated based on the weight of the control group (100) which were bred with the anticoccidiosis agent free feed and not inoculated.

(ii) Oocyst index

The number of oocyst in the caecum was counted on 8 days after the inoculation by homogenizing the intestinal canal. The oocyst index was defined as follows:

| The number of oocyst found in the intestinal canal | The oocyst index |
| --- | --- |
| $0.0 - 0.1 \times 10^6$ | 0 |
| $0.1 - 1.0 \times 10^6$ | 1 |
| $1.0 - 5.0 \times 10^6$ | 10 |
| $5.0 - 11.0 \times 10^6$ | 20 |
| $>11.0 \times 10^6$ | 40 |

(iii) Disease index of the intestinal canal

The chickens tested were anatomized at the end of the test (8 days after the fraction), and the intestinal canal was examined with the naked eye to determine the disease index. The disease index was defined as follows and the disease value was defined ten times as many as the value of the disease index.

| | |
| --- | --- |
| 0, (−) | The caecum is quite normal. If bleeding spot is found, (−) is changed to (+). |
| 1, (+) | The caecum is normal in shape. The content therein is slightly fluid and yellowish. Slight swelling is found partly on a mucous membrane of the caecum which becomes whitish. |
| 2, (++) | The caecum is generally normal in shape. Swelling is found on the whole surface of a mucous membrane. No bleeding is found in the content. Mucus is slightly yellowish and faded. A few white spot-like necroses or bleeding spots are found in a mucous membrane. |
| 3, (+++) | The caecum is clearly withered and changed in shape, and is a little longer than the rectum. The content is quite abnormal and is often filled with coagulated blood or white-gray, cheese-like degenerated matter. The wall of the caecum is clearly swelled and easily broken and sometimes bleeding spots still remain. The disease reaches a basis of the caecum but not the rectum. |
| 4, (++++) | Withering and deformation of the caecum are remarkable. The caecum looks like a sausage in shape and is not longer than the rectum. The disease reaches almost one third or fourth of the rectum. |

Groups 1 to 3 were fed with the feed containing a predetermined concentration of the antibiotic 6270 from two days prior to the inoculation of oocyst. Group 4 was fed with the feed containing the antibiotic Monensin as a comparative agent. Group 5 is the infected non-treated control group inoculated with oocyst and fed with the basic feed containing no anticoccidiosis agent. Group 6 is a non-infected, non-treated control group.

TABLE 2

| Group | Antibiotic | Dose (ppm) | Weight increase (%) | Degree of bloody excrement* | | | | | Survival rate (%) | Average number of oocyst in the intestinal canal | Oocyst index | Disease index of the intestinal canal | | | | | Disease value | A.C.I. |
|---|---|---|---|---|---|---|---|---|---|---|---|---|---|---|---|---|---|---|
| | | | | 4 days | 5 days | 6 days | 7 days | 8 days | | | | +++ | ++++ | ++ | + | − | | |
| 1 | 6270 | 6.3 | 95.0 | + | +++ | +++ | + | − | 100 | $2.52 \times 10^6$ | 10 | 0 | 5 | 0 | 0 | 0 | 30 | 155.0 |
| 2 | 6270 | 12.5 | 98.6 | − | ++ | + | − | − | 100 | $2.40 \times 10^6$ | 10 | 0 | 0 | 2 | 3 | 0 | 14 | 174.6 |
| 3 | 6270 | 25 | 103.0 | − | − | − | − | − | 100 | $1.4 \times 10^3$ | 0 | 0 | 0 | 0 | 3 | 2 | 6 | 197.0 |
| 4 | Monensin | 80 | 101.4 | + | ++ | ++ | + | − | 100 | $7.03 \times 10^6$ | 20 | 0 | 1 | 4 | 0 | 0 | 22 | 159.4 |
| 5 | Infected, non-treated group | 0 | 67.7 | ++ | ++++ | ++++ | + | − | 100 | $7.27 \times 10^6$ | 20 | 5 | 0 | 0 | 0 | 0 | 40 | 107.7 |
| 6 | Non-infected, non-treated group | 0 | 100.0 | − | − | − | − | − | 100 | 0 | 0 | 0 | 0 | 0 | 0 | 5 | 0 | 200.0 |

* Degree of bloody excrement:  −: No bloody excrement
+: Less than 10%
++: 10—30%
+++: 30—50%
++++: More than 50%

EP 0 158 179 B1

From these test results, it is evident that the antibiotic 6270 has an anticoccidiosis activity.

Test Example 2: Feed efficiency test on swine
Pigs used: Land race species
Basic feed:
a) from the initiation of the test to four weeks later
    60% of grain (corn, wheat, barley), 15% of soybean cake, 15% of animal-based feed (skimmilk powder, fish powder), and 10% of others (enzyme, calcium carbonate, potassium phosphate, sodium chloride, etc).

b) from 4 to 12 weeks
    78% of grain (corn, miro, wheat), 13% of soybean cake, 5% fish powder and 4% of others (calcium carbonate, calcium phosphate, sodium chloride, etc.)

Method:
    Fifteen young pigs of 35 days old in an average were divided into three groups, each group consisting of five pigs, so that the average weight would be substantially equal. The antibiotic 6270 was added to the basic feed in an amount of 0, 12.5 and 25 ppm, respectively. And then the three groups of pigs were fed with the feeds, respectively, for 12 weeks. Then, the body weight and the feed consumption were measured.
    Further, the overall number of days in which the pigs suffered from diarrhoea during the period of four weeks from the initiation of the test, were counted. The results are shown in Table 3.

TABLE 3

| Group | Amount of antibiotic 6270 added to the feed (ppm) | Average weight at the initiation of the test (kg) | Average weight increase during the period (kg) | Average amount of feed ingested during the test period (kg) | Feed demand index* | Improvement of the feed demand index (%)** | Overall number of days in which each group suffered from diarrhoea/ overall number of days during the test for each group |
|---|---|---|---|---|---|---|---|
| 1 | 12.5 | 8.20 | 45.82 | 106.9 | 2.333 | 5.0 | 13/140 |
| 2 | 25 | 8.18 | 48.30 | 110.1 | 2.280 | 7.2 | 5/140 |
| 3 | 0 | 8.23 | 43.73 | 107.4 | 2.456 | — | 48/140 |

$$* \text{ Feed demand index} = \frac{\text{Average amount of feed ingested during the test period}}{\text{Average weight increase during the test period}}$$

$$** \text{ Improvement in the feed demand index (\%)} = (1 - \frac{\text{Feed demand index when 6267 was added}}{\text{Feed demand index when no antibiotic was added}}) \times 100$$

The concentrated feed demand index was improved by from 7 to 10% by feeding the feed containing the antibiotic 6270. The weight increase was improved by from 9 to 11%.

The feed demand index was improved by from 5 to 8% by feeding the feed containing the antibiotic 6270. The weight increase was improved by from 5 to 11%. Further, it was observed that diarrhoea of pigs was substantially reduced.

Test Example 3: Feed efficiency of ruminant
Steers used: Holstein
Concentrated feed:

71.5% of grain (corn, miro, barley), 11.5% of chaff and bran (corn gluten feed, wheat bran, rice bran oil cake), 5.5% of vegetable oil cake (soybean cake, linseed oil cake), and 11.5% of others (alphalpha meal, molasses, calcium phosphate, sodium chloride)

Method:

Fifteen castrated steers of 8 months old were divided into three groups, each consisting of five steers, so that the average body weight will be substantially equal. The antibiotic 6270 was added to the basic concentrated feed in an amount of 0, 7.5 and 15 ppm, respectively. The three groups of steers were fed with the feeds, respectively. Further, dried rice straw was fed as crude feed in an amount of 1 kg per steer. The results are shown in Table 4.

TABLE 4

| Group | Amount of antibiotic 6270 added to the feed (ppm) | Average weight at the initiation of the test (kg) | Average weight increase during the period (kg) | Average amount of concentrated feed ingested during the test period (kg) | Concentrated feed demand index* | Improvement of the concentrated feed demand index (%)** |
|---|---|---|---|---|---|---|
| 1 | 7.5 | 320 | 308 | 2310 | 7.500 | 9.2 |
| 2 | 15 | 318 | 310 | 2360 | 7.613 | 7.8 |
| 3 | 0 | 323 | 281 | 2320 | 8.256 | — |

$$* \text{ Concentrated feed demand index} = \frac{\text{Average amount of feed ingested during the test period}}{\text{Average weight increase during the test period}}$$

$$** \text{ Improvement of the concentrated feed demand index (\%)} = \left(1 - \frac{\text{Feed demand index when 6267 was added}}{\text{Feed demand index when no antibiotic was added}}\right) \times 100$$

EP 0 158 179 B1

## Claims

1. An antibiotic 6270 having the following physicochemical properties and a salt thereof:
   (1) Colorless needle-like crystals; acidic substance
   (2) Melting point: 115—118°C
   (3) Elementary analysis (Found %): C: 62.72%, H: 9.49%; O: 27.78%
   (4) Specific rotation: $[\alpha]_D^{20}$—11.5° (C 1.0, methanol)
   (5) Ultraviolet absorption spectrum: The maximum absorption bands not observed at 210 nm or more, as measured in the methanol solution
   (6) Characteristic absorption (cm$^{-1}$) in the infrared absorption spectrum (taken with the potassium bromide tablet): 3470, 2980, 2945, 2890, 1735, 1460, 1380, 1315, 1165, 1102, 1075, 987, 980.
   (7) Solubility: Soluble in methanol ethanol, ethyl acetate, chloroform, ether, acetone, and benzene; insoluble in water.
   (8) Color reactions: Positive in the vanilline-sulfuric acid reaction and the Dragendorff reaction; negative in the ninhydrin reaction; colors with $I_2$ gas.
   (9) Thin layer chromatography (by means of Kieselgel GF$_{254}$, manufactured by Merck Co.):

| Solvent system | Rf value |
| --- | --- |
| Chloroform-methanol (20:1) | 0.49 |
| n-Hexane-ethyl acetate (1:1) | 0.08 |
| Benzene-ethyl acetate (1:1) | 0.04 |
| Benzene-acetone (1:1) | 0.53 |
| Chloroform-acetone (2:1) | 0.36 |
| n-Hexane-ethyl acetate (1:3) | 0.13 |
| Ethyl acetate | 0.22 |

   (10) $^1$H-NMR spectrum: The presence of three methoxy groups was observed at δppm 3.39 (3H), 3.49 (6H), as measured at 100 MHz in heavy chloroform by using TMS as internal standard;
   (11) $^{13}$C-NMR spectrum: the total number of carbon atoms is 44, as measured at 25 MHz in heavy chloroform by using TMS as internal standard;
   (12) The antibacterial spectrum is shown in Table 1 of the description.

2. A process for producing the antibiotic 6270 as defined in Claim 1, which comprises culturing an antibiotic 6270-producing microorganism belonging to the genus *Nocardiopsis* and isolating the antibiotic 6270 from the culture product.

3. The process according to Claim 2, wherein the microorganism is FERM BP-717.

4. An anticoccidiosis agent comprising the antibiotic 6270 as defined in Claim 1, as an effective ingredient.

5. A method for prevention and cure of coccidiosis of domestic fowls and animals which comprises administering an effective amount of the antibiotic 6270 as defined in Claim 1 to the domestic fowls and animals.

6. A growth accelerating and feed efficiency increasing agent for domestic animals and fowls comprising the antibiotic 6270 as defined in Claim 1 as an effective ingredient.

7. A feed of domestic fowls and animals comprising the antibiotic 6270 as defined in Claim 1 in an amount effective against coccidiosis of the fowls and animals.

8. The feed of Claim 7, wherein the antibiotic 6270 is contained in a concentration of about 0.5 to 200 ppm.

9. A feed of domestic animals or fowls comprising the antibiotic 6270 as defined in Claim 1 in an amount effective in accelerating the growth of the animals or fowls and the increase in feed efficiency.

10. The feed of Claim 9, wherein the antibiotic 6270 is contained in a concentration of about 0.5 to 200 ppm.

11. The microorganism *Nocardiopsis* sp. 6270, FERM BP-717.

## Patentansprüche

1. Antibiotikum 6270 mit den folgenden physikochemischen Eigenschaften und ein Salz desselben:
   (1) farblose, nadelartige Kristalle; saure Substanz
   (2) Schmelzpunkt: 115—118°C
   (3) Elementaranalyse (gefunden %) C: 62,72% H: 9,49% O: 27,78%
   (4) Spezifische Drehung: $[\alpha]_D^{20}$—11,5° (C 1,0, Methanol)
   (5) UV-Absorptionsspektrum: Die Maximumabsorptionsbanden nicht beobachtet bei 210 nm oder mehr, bei Messungen in der Methanollösung
   (6) Charakteristische Absorption (cm$^{-1}$) im Infrarotabsorptionsspektrum (aufgenommen mit der Kaliumbromid-Tablette): 3470, 2980, 2945, 2890, 1735, 1460, 1380, 1315, 1165, 1102, 1075, 987, 980.
   (7) Löslichkeit: Löslich in Methanol, Ethanol, Ethylacetat, Chloroform, Ether, Aceton und Benzol; unlöslich in Wasser.

(8) Farbreaktionen: Positiv bei der Vanillin-Schwefelsäure-Reaktion und der Dragendorff-Reaktion; Negativ bei der Ninhydrin-Reaktion; färbt sich mit $J_2$ Gas.

(9) Dünnschicht-Chromatographie (mittels Kieselgel $GF_{254}$, hergestellt von MERCK Co.):

| Lösungsmittelsystem | RF-Wert |
|---|---|
| Chloroform-Methanol (20:1) | 0,49 |
| n-Hexan-Ethylacetat (1:1) | 0,08 |
| Benzol-Ethylacetat (1:1) | 0,04 |
| Benzol-Aceton (1:1) | 0,53 |
| Chloroform-Aceton (2:1) | 0,36 |
| n-Hexan-Ethylacetat (1:3) | 0,13 |
| Ethylacetat | 0,22 |

(10) $^1$H-NMR-Spektrum: Die Anwesenheit von drei Methoxy-Gruppen wird beobachtet bei ppm 3,39 (3H), 3,49 (6H), gemessen bei 100 MHz in schwerem Chloroform unter Verwendung von TMS als internem Standard;

(11) $^{13}$C-NMR-Spektrum: die Gesamtzahl der Kohlenstoffatome beträgt 44, gemessen bei 25 MHz in schwerem Chloroform unter Verwendung von TMS als internem Standard;

(12) Das antibakterielle Spektrum ist in Tabelle 1 der Beschreibung gezeigt.

2. Verfahren zur Herstellung des Antibiotikum 6270 wie in Anspruch 1 definiert, umfassend die Kultivierung eines Antibiotikum 6270-produzierenden Mikroorganismus, der zur Gattung *Nocardiopsis* gehört und Isolierung des Antibiotikum 6270 aus dem Kulturprodukt.

3. Verfahren gemäß Anspruch 2, wobei der Mikroorganismus FERM BP-717 ist.

4. Anticocczidiosis Mittel, umfassend das Antibiotikum 6270, wie in Anspruch 1 definiert, als einen wirksamen Bestandteil.

5. Verfahren zur Verhinderung und Heilung von Cocczidiosis bei Hausgeflügel und Tieren, umfassend die Verabreichung einer wirksamen Menge des Antibiotikums 6270, wie in Anspruch 1 definiert, an das Hausgeflügel und die Tiere.

6. Mittel zur Wachstumsbeschleunigung und zur Steigerung der Futtereffizienz für Haustiere und Geflügel, umfassend das Antibiotikum 6270, wie in Anspruch 1 definiert, als einen wirksamen Bestandteil.

7. Futter für Hausgeflügel und Tiere, umfassend das Antibiotikum 6270, wie in Anspruch 1 definiert, in einer Menge, die wirksam ist gegen Cocczidiosis bei dem Geflügel und den Tieren.

8. Futter gemäß Anspruch 7, wobei das Antibiotikum 6270 in einer Konzentration von etwa 0,5 bis 200 ppm enthalten ist.

9. Futter für Haustiere oder Geflügel, umfassend das Antibiotikum 6270, wie in Anspruch 1 definiert, in einer Menge, die wirksam ist bei der Beschleunigung des Wachstums der Tiere oder des Geflügels und der Steigerung der Futtereffizienz.

10. Futter gemäß Anspruch 9, wobei das Antibiotikum 6270 in einer Konzentration von etwa 0,5 bis 200 ppm enthalten ist.

11. Mikroorganismus *Nocardiopsis* sp. 6270, FERM BP-717.

**Revendications**

1. Un antibiotique 6270 ayant les propriétés physico-chimiques suivantes et un sel de ce dernier:

(1) Cristaux incolores de type aiguilles; substance acide

(2) Point de fusion: 115—118°C

(3) Analyse élémentaire (Trouvé %): C: 62,72%, H: 9,49%; O: 27,78%

(4) Rotation spécifique: $[\alpha]_D^{20}$—11,5° (C 1,0, méthanol)

(5) Spectre d'absorption ultraviolet: Les bandes d'absorption maximale non observées à 210 nm ou plus, tel que mesuré dans la solution de méthanol

(6) Absorption caractéristique ($cm^{-1}$) dans le spectre d'absorption infrarouge (pris avec la pastille de bromure de potassium): 3470, 2980, 2945, 2890, 1735, 1460, 1380, 1315, 1165, 1102, 1075, 987, 980

(7) Solubilité: soluble dans le méthanol, l'éthanol, l'acétate d'éthyle, le chloroforme, l'éther, l'acétone et le benzène; insoluble dans l'eau

(8) Réactions colorées: positives dans la réaction à la vanilline-acide sulfurique et dans la réaction de Dragendorff; négatives dans la réaction à la Ninhydrine; couleurs avec $I_2$ gazeux

(9) Chromatographie sur couche mince (au moyen de Kieselgel $GF_{254}$, fabriqué par Merck Co.):

| Système solvant | Valeur de Rf |
|---|---|
| Chloroforme-méthanol (20:1) | 0,49 |
| n-Hexane-acétate d'éthyle (1:1) | 0,08 |
| Benzène-acétate d'éthyle (1:1) | 0,04 |
| Benzène-acétone (1:1) | 0,53 |
| Chloroforme-acétone (2:1) | 0,36 |
| n-Hexane-acétate d'éthyle (1:3) | 0,13 |
| Acétate d'éthyle | 0,22 |

(10) Spectre RMN $^1$H: la présence de trois groupes méthoxy a été observée à δppm 3,39 (3H), 3,49 (6H), tel que mesuré à 100 MHz dans du chloroforme lourd à l'aide du TMS comme étalon interne;

(11) Spectre de RMN[13]C: le nombre total d'atomes de carbone est de 44, tel que mesuré à 25 MHz dans du chloroforme lourd à l'aide du TMS comme étalon interne;

(12) Le spectre antibactérien est représenté dans le Tableau 1 de la description.

2. Procédé de fabrication de l'antibiotique 6270 tel que défini à la revendication 1, qui comprend la culture d'un microorganisme producteur de l'antibiotique 6270 appartenant au genre *Nocardiopsis* et l'isolement de l'antibiotique 6270 à partir du produit de culture.

3. Procédé selon la revendication 2, dans lequel le microorganisme est FERM BP-717.

4. Agent anticoccidiose comprenant l'antibiotique 6270 tel que défini à la revendication 1, en tant qu'ingrédient efficace.

5. Procédé pour prévenir et soigner la coccidiose de volailles et d'animaux domestiques, qui comprend l'administration d'une quantité efficace de l'antibiotique 6270 tel que défini à la revendication 1 aux volailles et animaux domestiques.

6. Agent accélérant la croissance et augmentant l'efficacité des aliments chez les animaux et volailles domestiques, comprenant l'antibiotique 6270 tel que défini à la revendication 1, en tant qu'ingrédient efficace.

7. Aliment pour volailles et animaux domestiques comprenant l'antibiotique 6270 tel que défini à la revendication 1, dans une quantité efficace pour lutter contre la coccidiose des volailles et des animaux.

8. Aliment selon la revendication 7, dans lequel l'antibiotique 6270 est contenu dans une concentration d'environ 0,5 à 200 ppm.

9. Aliment pour animaux ou volailles domestiques comprenant l'antibiotique 6270 tel que défini à la revendication 1 dans une quantité efficace pour accélérer la croissance des animaux ou volailles et pour augmenter l'efficacité des aliments.

10. Aliment selon la revendication 9, dans lequel l'antibiotique 6270 est contenu dans une concentration d'environ 0,5 à 200 ppm.

11. Microorganisme *Nocardiopsis* sp. 6270, FERM BP-717.

FIGURE I

wave number cm⁻¹

FIGURE 2

EP 0 158 179 B1

FIGURE 3